# FASCICULE DE BREVET EUROPEEN

(11) **EP 1 883 799 B1**
(45) Date de publication et mention de la délivrance du brevet: **05.10.2011**
(21) Numéro de dépôt: 06764660.4
(22) Date de dépôt: 22.05.2006
(51) Int. Cl.: G01N 3/06, G01B 5/30, G01B 5/02, G01N 3/08, G01N 33/24

(54) **COLLIER DE MESURE DE LA DÉFORMATION LATÉRALE D'UNE ÉPROUVETTE LORS D'ESSAIS DE COMPRESSION, NOTAMMENT UNIAXIALE OU TRIAXIALE**
RING ZUR MESSUNG DER SEITLICHEN VERFORMUNG EINES PRÜFLINGS IN UNIAXIALEN ODER TRIAXIALEN KOMPRESSIONSTESTS
COLLAR FOR MEASURING THE LATERAL DEFORMATION OF A TEST PIECE DURING COMPRESSION TESTS, SUCH AS UNIAXIAL OR TRIAXIAL COMPRESSION TESTS

(30) Priorité: 24.05.2005 FR 0505204
(43) Date de publication de la demande: 06.02.2008
(73) Titulaire: UNIVERSITE DES SCIENCES ET TECHNOLOGIES DE LILLE, 59655 Villeneuve d'Ascq (FR)
(72) Inventeur: SECQ, Jean Univ. des Sciences et Techn. de Lille, 59655 Villeneuve D'Asq (FR)
(74) Mandataire: Boubal, Denis Henri Jacques
(86) Numéro de dépôt international: PCT/FR2006/001164
(87) Numéro de publication internationale: WO 2006/125903

(56) Documents cités:
- FR-A- 2 566 531
- FR-A- 2 663 121
- FR-A- 2 722 565
- US-A- 3 779 085
- US-A- 4 047 425
- US-A- 4 905 521
- US-A- 5 483 836

## Description

L'invention se rapporte à un dispositif de mesure de la déformation latérale d'une éprouvette lors d'essais de compression, notamment uniaxiale ou triaxiale.

Elle trouvera une application particulière dans une cellule triaxiale polyvalente d'essais pour géomatériaux d'échantillons de roche, de sol, de matériaux cimentaires ou de matériaux fabriqués, sur les sites de prélèvement, dans des conditions d'essais des laboratoires.

Ces échantillons, encore appelés éprouvettes, de forme cylindrique, peuvent être soumis à différentes conditions de pression, de chargement, de température, et de drainage, ces paramètres pouvant être contrôlés à l'aide de capteurs de pression, de température, de déplacement interne ou externe.

Les contraintes de pression peuvent être dirigées axialement et/ou sur la face latérale de l'échantillon.

On connaît du document FR-2.566.531 un capteur de déplacement latéral, se présentant sous la forme d'un collier, constitué par une pluralité de rouleaux parallèles, articulés entre eux, et aptes à enserrer l'échantillon maintenu par un système à ressort. Ce collier permet de mesurer la déformation latérale de l'échantillon en mesurant l'écartement des deux derniers rouleaux.

On connaît du document FR-2.663.121 une cellule triaxiale d'essais polyvalente pour géomatériaux. Cette cellule comprend une chambre de pression à l'intérieur de laquelle est placée l'éprouvette cylindrique. Elle comprend au moins un vérin de compression apte à exercer une poussée uniaxiale longitudinalement à l'éprouvette.

Une pression latérale, et plus particulièrement radiale, est exercée sur l'éprouvette en soumettant la face latérale de ladite éprouvette cylindrique à la pression d'un fluide. Un système hydraulique de compensation permet par ailleurs d'équilibrer les efforts longitudinaux et radiaux.

Aussi, l'échantillon est immergé dans un fluide, tel que de l'huile, et est protégé dudit fluide au moyen d'un fourreau se présentant sous la forme d'un manchon constitué par une membrane élastique. Afin de mesurer la déformation latérale de l'échantillon, un collier du type connu, constitué de rouleaux, est placé autour du fourreau en le ceinturant.

Néanmoins, un tel collier s'avère peu satisfaisant, car ces rouleaux ont tendance à s'imprimer dans la paroi élastique de la membrane, entravant ainsi leur déplacement par roulement autour de la périphérie du manchon.

En outre, il a été constaté que l'élasticité dé la paroi de la membrane fausse la mesure, le collier mesurant la déformation latérale de l'échantillon, mais également la déformation latérale de ladite membrane.

Par ailleurs, le collier connu précité, constitué de rouleaux, s'avère relativement encombrant et nécessite une cellule d'essais dont la chambre de pression est de dimension suffisamment grande, notamment en largeur, pour pouvoir être installée.

On connaît du document FR-2.722.565 un dispositif de mesure des variations de la longueur d'une éprouvette, constitué par un anneau ouvert dont les extrémités libres sont munies de couteaux destinés respectivement à s'appuyer sur deux points de l'éprouvette. Ce dispositif permet de mesurer la déformation de l'échantillon entre ces deux points.

On connaît du document US-4.047.425 un dispositif pour la mesure latérale de la pression induite par une éprouvette lors d'essais de compression verticale, mais ne concerne pas les essais de compression triaxiale.

On connaît du document US-4.905.521 un dispositif permettant la mesure de la déformation latérale d'un échantillon, plus particulièrement lors d'une compression triaxiale. Ce dispositif comprend un collier de mesure constitué par une chaîne, permettant une mesure globale de la déformation latérale de l'échantillon, qui coopère avec un fourreau en élastomère qui épouse la paroi latérale de l'échantillon afin de le protéger. Ce dispositif est conforme au préambule de la revendication 1.

On connaît du document US-3.779.085 un dispositif de mesure de l'expansion du béton.

On connaît du document US-5.483.836 un dispositif permettant la mesure de la déformation latérale d'une éprouvette entre deux points. Il est constitué par un collier à bascule comprenant deux segments en arc de cercle, articulés l'un à l'autre. Une jauge de déformation permet la mesure de la rotation d'un segment par rapport à l'autre et ainsi la mesure de déformation de l'échantillon entre deux points de contact.

Le but de la présente invention est de proposer un collier de mesure de la déformation latérale d'une éprouvette qui pallie les inconvénients précités, permettant ainsi d'augmenter la précision des mesures.

Un autre but de l'invention est de proposer un collier de mesure simple de réalisation et à faible coût de revient.

Un autre but de l'invention est de proposer un fourreau de protection pour éprouvette spécifique, adapté pour être utilisé avec un collier conforme à l'invention.

D'autres buts et avantages de la présente invention apparaîtront au cours de la description qui va suivre, qui n'est cependant donnée qu'à titre indicatif, et qui n'a pas pour but de la limiter

L'invention concerne tout d'abord un dispositif comprenant un collier de mesure de déformation latérale d'une éprouvette, lors d'essais de compression triaxiale et un fourreau destiné à être utilisé dans une cellule d'essais, afin de protéger l'échantillon, et coopérant avec le collier, constitué par un manchon élastique, caractérisé en ce que le collier est constitué par un anneau métallique ou un anneau en matériaux composites, apte à enserrer ladite éprouvette en la ceinturant, monobloc, ouvert, et dont les extrémités libres sont écartées d'une distance Δ, ledit collier présentant en outre des moyens de mesure de l'écartement Δ desdites extrémités libres de l'anneau constitués, d'une part, par une lame élastique équipée d'au moins une jauge de contrainte, ladite lame étant précontrainte et assujettie à l'une des extrémités libres de l'anneau, et d'autre part, d'un doigt de contact assujetti à l'autre extrémité libre de l'anneau et apte à provoquer le fléchissement de la lame élastique lors de la déformation du collier, le doigt de contact étant disposé sensiblement perpendiculairement à la lame élastique, le collier présente en outre des premiers moyens de réglage de la position du doigt de contact par rapport à ladite lame élastique, ledit manchon présentant dans sa paroi au moins deux points durs régulièrement répartis sur la circonférence du manchon pour constituer des points d'appui par ledit collier, chaque dit point dur étant constitué par un insert d'épaisseur sensiblement égale à l'épaisseur de la paroi du manchon et affleurant des deux côtés de ladite paroi, l'insert présentant en outre des moyens d'enchevêtrement pour le maintien dudit insert au manchon.

L'invention sera mieux comprise à la lecture de la description suivante accompagnée des dessins en annexe parmi lesquels ;
- la figure 1 est une vue en perspective d'un collier de mesure conforme à l'invention,
- la figure 2 est une vue de dessus schématique du collier de mesure tel qu'illustré à la figure 1,
- la figure 3 est une vue en perspective d'un fourreau de protection conforme à l'invention,
- la figure 4 est une vue selon la coupe horizontale IV-IV telle qu'illustrée à la figure 3,
- la figure 5 est une vue de détail telle qu'illustrée au cadre V-V.

L'invention concerne tout d'abord un collier de mesure de la déformation latérale d'une éprouvette lors d'essais de compression, notamment uniaxiale ou triaxial.

On entend par déformation latérale, une déformation de l'éprouvette dans un plan perpendiculaire à l'axe longitudinal de l'éprouvette cylindrique.

Selon l'invention, le collier 1 est constitué par un anneau 2 métallique ou un anneau en matériaux composites, apte à enserrer ladite éprouvette. L'anneau 2 est ouvert. Les extrémités libres 3 de l'anneau sont écartées d'une distance Δ. Ledit collier 1 présente en outre des moyens 5 de mesure directement ou indirectement, de l'écartement Δ, desdites extrémités libres 3 de l'anneau 2, moyens constitués par au moins une jauge de contrainte 6.

La jauge de contrainte, encore appelée jauge extensométrique est un appareil permettant de suivre la déformation de matériaux soumis à des contraintes, au moyen de variations de résistance d'un conducteur électrique.

L'anneau métallique ou en matériaux composites est monobloc. Il permet d'enserrer l'éprouvette en la ceinturant.

Selon un mode de réalisation, les moyens 5 de mesure de l'écartement desdites extrémités libres de l'anneau sont constitués par quatre jauges de contrainte.

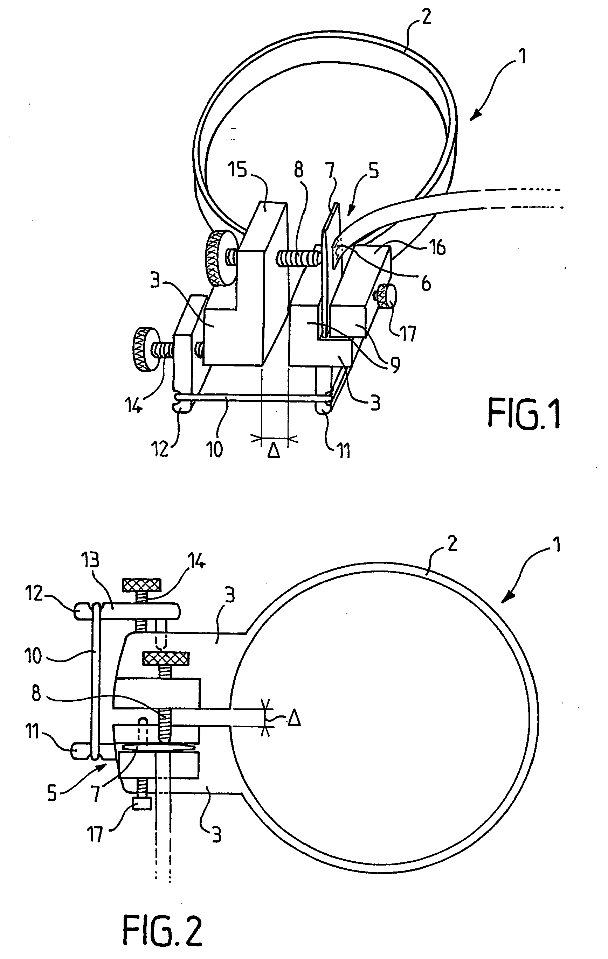

Selon un mode de réalisation particulier, illustré à la figure 1 ou 2, les moyens de mesure 5 sont constitués, d'une part, par une lame élastique 7 équipée de ladite au moins une jauge de contrainte 6, ladite lame élastique 7 étant précontrainte et assujettie à l'une des extrémités libres 3 de l'anneau 2, et d'autre part, d'un doigt de contact 8, assujetti à l'autre extrémité de l'anneau, et apte à provoquer le fléchissement de la lame élastique lors de la déformation du collier.

Les extrémités libres de l'anneau 3 sont constituées chacune par une protubérance de matière, sensiblement parallèles l'une à l'autre.

Telle qu'illustrée à la figure 1 ou 2, ladite lame élastique 7 est disposée à une extrémité 3 du collier 1, maintenue sensiblement perpendiculaire au plan du collier, par l'une de ses extrémités.

La ou les jauges de contrainte sont instrumentées, selon une technique connue, avec un dispositif de mesure électrique, apte notamment à mesurer les variations de résistance de la jauge. Ce dispositif, après étalonnage permet ainsi de retrouver la déformation de l'éprouvette.

Avantageusement, la lame élastique est précontrainte, c'est-à-dire sollicitée même lorsque le collier est au repos, afin que les valeurs mesurées par le dispositif de mesure électrique soient fiables et continues.

La ou les jauges de contrainte peuvent être assujetties à la lame élastique par collage.

Selon un mode de réalisation, le doigt de contact 8 est disposé sensiblement perpendiculairement à la lame élastique 7, le collier présentant en outre des premiers moyens de réglage de la position du doigt de contact par rapport à ladite lame élastique.

Tel qu'illustré aux figures 1 et 2, le doigt de contact 8 est fileté, se présentant notamment sous la forme d'une vis, et apte à coopérer avec le taraudage d'un alésage réalisé à l'une des extrémités libres 3 de l'anneau 2.

Le doigt de contact 8 est alors vissé, monté traversant au niveau d'un flanc 15 s'élevant d'une dite protubérance formant une dite extrémité libre 3.

Selon un mode de réalisation, l'une des extrémités libres 3 de l'anneau 2 présente une mâchoire de serrage 9, apte à maintenir ladite lame élastique 7.

Telle qu'illustrée à la figure 1, unedite protubérance de matière, formant unedite extrémité libre 3, présente un épaulement contre lequel un mors 16 vient serrer la lame élastique 7, au niveau de son extrémité basse, notamment au moyen d'une vis de serrage 17.

Un capot (non illustré) peut être prévu pour protéger la lame élastique 7. Le capot peut être prévu amovible, notamment assujetti au mors 16 de la mâchoire de serrage 9.

Le capot s'élève sur toute la hauteur de la lame 7 et présente un volume interne à l'intérieur duquel la lame 7 peut s'enserrer lors de la déformation du collier.

Selon un mode de réalisation, les extrémités libres 3 de l'anneau 2 sont reliées par des moyens élastiques 10 en extension. Les moyens élastiques 10 permettent avantageusement de précontraindre la lame élastique 7. Le collier peut présenter en outre des moyens de réglage de la force de rappel des moyens élastiques 10.

Les moyens élastiques 10 peuvent notamment prendre la forme d'un ressort en extension ou encore la forme d'un joint torique.

Les deux extrémités libres 3 de l'anneau 2 peuvent être reliées par les moyens élastiques 10 au niveau de deux points d'accroche 11, 12, le collier présentant en outre des seconds moyens de réglage 13, 14 de la position d'un point d'accroche 12 par rapport à l'une des extrémités libres 3 de l'anneau 2.

Lesdits seconds moyens de réglage sont illustrés par exemple aux figures 1 et 2. Pour faciliter leur illustration, lesdits seconds moyens de réglage ont été illustrés en dessous des extrémités libres 3 du collier à la figure 1 et dans le prolongement desdites extrémités libres 3 à la figure 2.

Aussi, avantageusement, un point d'accroche 13 est constitué par un élément 13 monté à coulissement, suivant la direction longitudinale des moyens élastiques, au niveau d'une dite extrémité libre 3 de l'anneau. Cet élément est taraudé, pour être traversé par une vis de positionnement 14, apte à faire appui sur un côté d'unedite extrémité libre 3.

Selon un mode de réalisation non illustré, les deux extrémités libres 3 de l'anneau sont, notamment, traversées par une tige de guidage, notamment au niveau d'un alésage traversant lesdites extrémités libres 3.

Cette tige de guidage, montée à coulissement au niveau d'au moins une dite extrémité libre 3, a pour fonction que le collier 1 se déforme de manière contrôlée, notamment sans torsion, afin que lesdites extrémités libres 3 restent maintenues sensiblement en vis-à-vis.

Cela étant, l'invention concerne également un fourreau 20, destiné à être utilisé dans une cellule d'essais, telle que par exemple celle décrite dans le document FR-2.663.121, afin de protéger une éprouvette 21, notamment minérale, de roche et/ou de sol, de béton, ledit fourreau 20 étant constitué par un manchon élastique 22, et apte à coopérer notamment avec un collier de mesure conforme à l'invention.

Selon l'invention, le manchon 22 présente dans sa paroi au moins deux points durs 24, régulièrement répartis sur la circonférence dudit manchon 22 et aptes à constituer des points d'appui pour ledit collier 1. Le manchon peut être réalisé à partir d'une membrane élastique notamment à partir d'un élastomère.

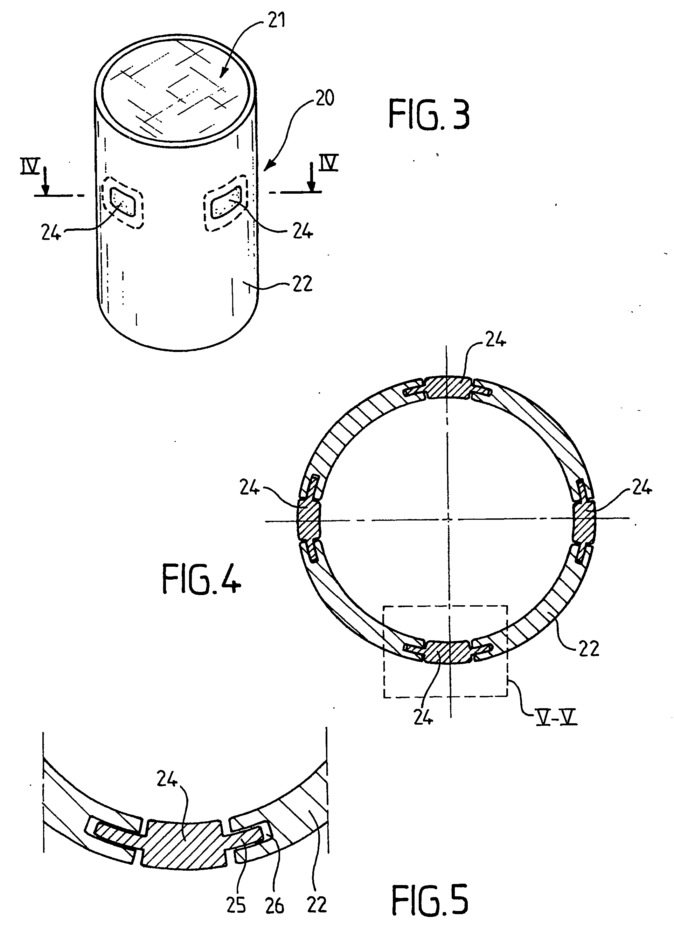

Tel qu'illustré à la figure 4, le manchon présente, selon un mode de réalisation, quatre points régulièrement répartis sur la circonférence dudit manchon à 90 °.

Ces points durs seront avantageusement constitués par un matériau présentant une rigidité supérieure à la membrane et une rigidité supérieure à l'échantillon de telle façon que leur déformation soit négligeable par rapport à la déformation de l'éprouvette. Ces points durs pourront être constitués notamment d'une matière métallique.

Selon un mode de réalisation, chaque point dur 24 est constitué par un insert, d'épaisseur sensiblement égale au moins à l'épaisseur de la paroi du manchon, et affleurant des deux côtés de ladite paroi. L'insert présente en outre des moyens d'enchevêtrement 25, 26 pour le maintien dudit insert au manchon.

Les moyens d'enchevêtrement pourront être constitués par un tenon latéral 25, apte à s'étendre, suivant la paroi du manchon, comme le montre la figure 4, à l'intérieur d'une mortaise 26 pratiquée dans ladite paroi de telle façon à constituer une chicane assurant l'étanchéité de la membrane au fluide.

Avantageusement, tel qu'illustré en pointillés à la figure 3, le tenon latéral est continu sur le pourtour de l'insert.

Ainsi, tel qu'illustré à la figure 5, lors de la déformation de la membrane, le tenon 25 est apte à glisser dans la mortaise 26 pratiquée dans la membrane assurant la fonction d'étanchéité même lors de déformations.

L'insert peut être avantageusement arqué d'un rayon de courbure sensiblement égal au rayon de courbure de la paroi du manchon. Avantageusement, le tenon latéral est avantageusement arqué de la même manière.

Le manchon peut être notamment réalisé en silicone. Le manchon est avantageusement surmoulé dans un moule où les inserts sont prépositionnés.

Par exemple, l'opération de moulage est réalisée sous vide pour prévenir l'apparition d'inclusions, notamment de bulles.

Naturellement, d'autres modes de réalisation, à la portée de l'homme de l'art, auraient pu être envisagés sans pour autant sortir du cadre de l'invention.

## Revendications

1. Dispositif comprenant un collier (1) de mesure de la déformation latérale d'une éprouvette, lors d'essais de compression triaxiale, et un fourreau (20) destiné à être utilisé dans une cellule d'essais, afin de protéger l'échantillon, et coopérant avec le collier, ledit fourreau étant constitué par un manchon élastique (22), **caractérisé en ce que** le collier (1) est constitué par un anneau (2) métallique ou un anneau en matériaux composites, apte à enserrer ladite éprouvette en la ceinturant, monobloc, ouvert, et dont les extrémités libres (3) sont écartées d'une distance Δ, ledit collier présentant en outre des moyens (5) de mesure de l'écartement Δ desdites extrémités libres (3) de l'anneau (2) constitués, d'une part, par une lame élastique (7) équipée d'au moins une jauge de contrainte (6), ladite lame élastique (7) étant précontrainte et assujettie à l'une des extrémités libres (3) de l'anneau (2), et d'autre part, d'un doigt de contact (8) assujetti à l'autre extrémité de l'anneau et apte à provoquer le fléchissement de la lame élastique (7) lors de la déformation du collier, le doigt de contact (8) étant disposé sensiblement perpendiculairement à la lame élastique (7), le collier présentant en outre des premiers moyens de réglage de la position du doigt de contact par rapport à ladite lame élastique, ledit manchon (22) présentant dans sa paroi au moins deux points durs (24) régulièrement répartis sur la circonférence du manchon pour constituer des points d'appui pour ledit collier, chaque dit point dur (24) étant constitué par un insert d'épaisseur sensiblement égale à l'épaisseur de la paroi du manchon (22) et affleurant des deux côtés de ladite paroi, l'insert présentant en outre des moyens d'enchevêtrement (25, 26) pour le maintien dudit insert au manchon (22).

2. Dispositif selon la revendication 1, dans lequel le doigt de contact (8) est fileté et apte à coopérer avec le taraudage d'un alésage réalisé à l'une des extrémités libres (3) de l'anneau (2).

3. Dispositif selon la revendication 1 ou 2, dans lequel l'une des extrémités libres (3) de l'anneau (2) présente une mâchoire de serrage (9) apte à maintenir ladite lame élastique (7).

4. Dispositif selon l'une des revendications 1 à 3, dans lequel les extrémités libres (3) de l'anneau (2) sont reliées par des moyens élastiques (10) en extension.

5. Dispositif selon la revendication 4 présentant en outre des moyens de réglage de la force de rappel des moyens élastiques.

6. Dispositif selon la revendication 5, dans lequel les deux extrémités libres (3) de l'anneau (2) sont reliées par les moyens élastiques (10) au niveau de deux points d'accroche (11, 12), le collier présentant en outre des seconds moyens de réglage (13, 14) de la position d'un point d'accroche (12) par rapport à l'une des extrémités libres (3) de l'anneau (2),

7. Dispositif selon la revendication 1, dans lequel les moyens d'enchevêtrement sont constitués par un tenon (25) latéral apte à s'étendre suivant la paroi du manchon à l'intérieur d'une mortaise (26) pratiquée dans ladite paroi de telle façon à constituer une chicane assurant l'étanchéité du manchon au fluide.

8. Dispositif selon la revendication 7, dans lequel l'insert est arqué d'un rayon de courbure sensiblement égal au rayon de courbure de la paroi du manchon (22).

## Claims

1. Device comprising a collar (1) for measuring the lateral deformation of a test piece, during triaxial compression tests, and a shaft (20) intended to be used in a test unit, in order to protect the sample, and cooperating with the collar, said shaft being comprised of an elastic sleeve (22), **characterised in that** the collar (1) comprises a metal hoop (2) or a hoop that is made from composite materials, which can clasp said test piece by surrounding it, which is a single piece, open, and of which the free ends (3) are separated by a distance Δ, said collar further having means (5) for measuring the separation Δ of said free ends (3) of the hoop (2) comprised, on the one hand, by a spring plate (7) provided with at least one strain gage (6), said spring plate (7) being pre-stressed and fixed to one of the free ends (3) of the hoop (2), and on the other hand, of a contact finger (8) fixed to the other end of the hoop and able to cause the bending of the spring plate (7) during the deformation of the collar, the contact finger (8) being arranged substantially perpendicularly to the spring plate (7), the collar further having a first means of adjustment of the position of the contact finger in relation to said spring plate, said sleeve (22) having in its wall at least two hard points (24) that are evenly spaced on the circumference of the sleeve in order to form support points for said collar, each said hard point (24) being comprised of an insert of a thickness that is substantially equal to the thickness of the wall of the sleeve (22) and flush with the two sides of said wall, the insert further having means for overlapping (25, 26) in order to maintain said insert to the sleeve (22).

2. Device according to claim 1, wherein the contact finger (8) is threaded and able to cooperate with the threading of a bore made in one of the free ends (3) of the hoop (2).

3. Device according to claim 1 or 2, wherein one of the free ends (3) of the hoop (2) has a clamping jaw (9) able to maintain said spring plate (7).

4. Device according to one of claims 1 to 3, wherein the free ends (3) of the hoop (2) are connected by elastic means (10) in extension.

5. Device according to claim 4 further having means of adjusting the return force of the spring means.

6. Device according to claim 5, wherein the two free ends (3) of the hoop (2) are connected by the spring means (10) on the two attaching points (11, 12), the collar further having second means of adjusting (13, 14) the position of an attaching point (12) in relation to one of the free ends (3) of the hoop (2).

7. Device according to claim 1, wherein the means for overlapping are comprised of a lateral tenon (25) able to extend according to the wall of the sleeve inside a mortice (26) made in said wall in such a way as to form a baffle providing the seal of the sleeve to the fluid.

8. Device according to claim 7, wherein the insert is arched by a radius of curvature that is substantially equal to the radius of curvature of the wall of the sleeve (22).

## Patentansprüche

1. Vorrichtung, umfassend eine Schelle (1) zum Messen der seitlichen Verformung eines Prüfkörpers bei dreiachsigen Kompressionsversuchen und eine Gleithülse (20), die dazu gedacht ist, in einer Prüfzelle verwendet zu werden, um den Prüfkörper zu schützen, und die mit der Schelle zusammenwirkt, wobei die Gleithülse aus einer elastischen Muffe (22) besteht, **dadurch gekennzeichnet, dass** die Schelle (1) aus einem einstückigen offenen Metallring (2) oder einem Ring aus Verbundmaterialien besteht, der dazu geeignet ist, den Prüfkörper festzuhalten, indem er ihn umklammert, und dessen freie Enden (3) um einen Abstand Δ entfernt sind, wobei die Schelle ferner Mittel (5) zum Messen der Entfernung Δ der freien Enden (3) des Rings (2) aufweist, die einerseits aus einem elastischen Plättchen (7), das mit mindestens einem Dehnungsmessgerät (6) ausgestattet ist, wobei das elastische Plättchen (7) vorgespannt ist und an einem der freien Enden (3) des Rings (2) befestigt ist, und andererseits aus einem Kontaktfinger (8), der an dem anderen Ende des Rings befestigt ist und dazu geeignet ist, die Biegung des elastischen Plättchens (7) bei der Verformung der Schelle zu verursachen, bestehen, wobei der Kontaktfinger (8) im Wesentlichen rechtwinklig zu dem elastischen Plättchen (7) angeordnet ist, wobei die Schelle ferner erste Mittel zum Einstellen der Position des Kontaktfingers im Verhältnis zu dem elastischen Plättchen aufweist, wobei die Muffe (22) in ihrer Wand mindestens zwei feste Stellen (24) aufweist, die gleichmäßig über dem Umfang der Muffe verteilt sind, um Stützpunkte für die Schelle zu bilden, wobei jede feste Stelle (24) aus einer Einlage besteht, die im Wesentlichen ebenso dick ist wie die Dicke der Wand der Muffe (22) und auf beiden Seiten der Wand bündig ist, wobei die Einlage ferner Verblockungsmittel (25, 26) zum Festhalten der Einlage an der Muffe (22) aufweist.

2. Vorrichtung nach Anspruch 1, wobei der Kontaktfinger (8) mit einem Gewinde versehen ist und dazu geeignet ist, mit dem Innengewinde einer Bohrung zusammenzuwirken, die an einem der freien Enden (3) des Rings (2) ausgebildet ist.

3. Vorrichtung nach Anspruch 1 oder 2, wobei eines der freien Enden (3) des Rings (2) eine Klemmbacke (9) aufweist, die dazu geeignet ist, um das elastische Plättchen (7) festzuhalten.

4. Vorrichtung nach einem der Ansprüche 1 bis 3, wobei die freien Enden (3) des Rings (2) über in Längung elastische Mittel verbunden sind.

5. Vorrichtung nach Anspruch 4, ferner aufweisend Mittel zum Einstellen der Rückstellkraft der elastischen Mittel.

6. Vorrichtung nach Anspruch 5, wobei die beiden freien Enden (3) des Rings (2) über elastische Mittel (10) an zwei Aufhängestellen (11, 12) verbunden sind, wobei die Schelle ferner zweite Mittel (13, 14) zum Einstellen der Position einer Aufhängestelle (12) im Verhältnis zu einem der freien Enden (3) des Rings (2) aufweist.

7. Vorrichtung nach Anspruch 1, wobei die Verblockungsmittel aus einem seitlichen Zapfen (25) bestehen, der dazu geeignet ist, um sich an der Wand der Muffe entlang im Innern eines Zapfenlochs (28) zu erstrecken, das in der Wand derart eingerichtet ist, dass es eine Schikane bildet, welche die Fluiddichte der Muffe sicherstellt.

8. Vorrichtung nach Anspruch 7, wobei die Einlage mit einem Krümmungsradius gebogen ist, der im Wesentlichen gleich dem Krümmungsradius der Wand der Muffe (22) ist.
